# EUROPÄISCHE PATENTSCHRIFT

(11) **EP 1 407 716 B1**
(45) Veröffentlichungstag und Bekanntmachung des Hinweises auf die Patenterteilung: **01.02.2006**
(21) Anmeldenummer: 03405725.7
(22) Anmeldetag: 09.10.2003
(51) Int. Cl.: A61B 17/11

(54) **Vorrichtung zur Durchführung einer Anastomose**
Device for performing anastomosis
Dispositif pour pratiquer une anastomose

(30) Priorität: 10.10.2002 CH 16972002
(43) Veröffentlichungstag der Anmeldung: 14.04.2004
(73) Patentinhaber: Zürcher Hochschule Winterthur, 8401 Winterthur (CH)
(72) Erfinder: Medricky, Peter, 8404 Winterthur (CH); Brom, Charles, 8400 Winterhur (CH)
(74) Vertreter: Wiedmer, Edwin

(56) Entgegenhaltungen:
- WO-A-01/91628
- US-A- 5 839 639
- US-A- 6 030 370

## Beschreibung

Die Erfindung betrifft eine Vorrichtung zur Durchführung einer Anastomose mit einer Nähverbindung zwischen einer Seitenwand oder einem endständigen Stumpf eines ersten Hohlorgans und einer Seitenwand oder einem endständigen Stumpf eines zweiten Hohlorgans.

Bei der Durchführung einer Koronaranastomose nach dem klassischen Operationsverfahren muss der Brustkorb durch eine Längsteilung des Brustbeins so weit geöffnet werden, dass der Operateur einen direkten Zugang zum Herz hat.

Heute sind auch mikrochirurgische Techniken für die mikroskopische Herzchirurgie in Gebrauch. Bekannt ist beispielsweise ein Telemanipulationssystem, bei dem der Operateur von einer Masterkonsole aus über zwei Instrumentengriffe den Eingriff steuert. Bewegungssensoren nehmen die Bewegungen des Operateurs an den Instrumentengriffen wahr und übertragen diese prozessgesteuert auf drei Manipulatoren, welche ein Videoskop sowie zwei auswechselbare endoskopische Instrumente führen. Die Instrumente verfügen an der Spitze über ein miniaturisiertes mechanisches Handgelenk, mit dem auch feinste Bewegungen, wie sie zum Nähen einer Koronaranastomose erforderlich sind, ermöglicht werden.

Ein wesentlicher Nachteil von Telemanipulationssystemen liegt darin, dass die Durchführung komplexer Bewegungen, wie sie etwa einem Nähvorgang zugrunde liegen, schwierig ist und vom Operateur zur präzisen Steuerung eines gewünschten Bewegungsablaufes über das Videoskop viel Übung erfordert. Der Chirurg muss den Nähvorgang ohne taktilen Feedback rein visuell selber durchführen.

Aus der US-A 6 030 370 ist ein chirurgisches Instrument zur Durchführung einer Gefässanastomose bekannt. Das Instrument zeichnet sich aus durch einen rohrförmigen, stirnseitig verschlossenen Schaft mit einem Aussendurchmesser, der die Einführung in die miteinander zu vernähenden Gefässe ermöglicht. In der Wand des Schaftes sind im Bereich des stirnseitigen Endes Saugöffnungen angeordnet und in Abstand zu dessen stirnseitigem Ende ist der Schaft mit einem Sauganschluss versehen. Nach dem Einführen des Schaftes in die miteinander zu vernähenden Gefässteile wird der Schaft an eine Saugquelle angeschlossen, so dass die Gefässteile im Nähbereich über die Saugöffnungen an den Schaft angesaugt und zur Durchführung des Nähvorganges fixiert werden.

Der Erfindung liegt die Aufgabe zugrunde, eine Vorrichtung der eingangs genannten Art zu schaffen, mit welcher die Herstellung der Nähverbindung automatisiert und beispielsweise von einem Roboter übernommen werden kann.

Die erfindungsgemässe Lösung der Aufgabe zeichnet sich aus durch
- einen ersten Halter mit einer diesen durchsetzenden, von einer Wand mit Saugöffnungen begrenzten Öffnung zur Aufnahme und Fixierung eines Seitenwandbereiches oder eines endständigen Stumpfes des ersten Hohlorgans und
- einen zweiten Halter mit einer diesen durchsetzenden, von einer Wand mit Saugöffnungen begrenzten Öffnung zur Aufnahme und Fixierung eines Seitenwandbereiches oder eines endständigen Stumpfes des zweiten Hohlorgans,
   wobei
- die Saugöffnungen mit einer Unterdruckquelle verbindbar sind,
- die Öffnungen auf je einer Seite der Halter eine im wesentlichen gleiche Kontur aufweisen,
- im Bereich der Ränder der Öffnungen mit der im wesentlichen gleichen Kontur Führungen für wenigstens ein Verbindungselement angeordnet sind und
- die beiden Halter so aneinander anlegbar sind, dass sich die Öffnungen mit der im wesentlichen gleichen Kontur zur Herstellung der Nähverbindung im wesentlichen deckungsgleich gegenüberstehen.

Ein wesentlicher Kern der Erfindung liegt darin, dass die beiden miteinander zu verbindenden Gefässabschnitte durch die Positionierung der zwei speziell ausgestalteten Halter definiert beschnitten werden, so dass sie nach dem Zusammenführen der beiden Halter für den anschliessenden Nähvorgang optimal aufeinanderliegen.

Mit der erfindungsgemässen Vorrichtung wird der Nähvorgang vollständig automatisierbar und kann dadurch reproduzierbar mit grosser Genauigkeit ausgeführt werden.

Bei einer ersten bevorzugten Ausführungsform der erfindungsgemässen Vorrichtung sind die Ränder der Öffnungen mit der im wesentlichen gleichen Kontur als umlaufende Nuten mit kreisbogenförmigem Querschnitt ausgebildet und die beiden Halter sind so aneinander anlegbar, dass sich die umlaufenden Nuten gegenüberstehen und eine Führungsnut für eine Helixnadel bilden.

Zur automatischen Ausführung einer Drehbewegung kann am rückwärtigen Ende der Helixnadel eine Achse zur Verbindung mit einem Antriebsaggregat festgelegt sein.

Die Helixnadel zeichnet sich dadurch aus, dass sie die Form einer Schraubenlinie mit konstantem Durchmesser und konstanter Steigung aufweist. Die Nadel eignet sich ganz allgemein zur Herstellung einer Verbindungsnaht im Medizinalbereich und kann zur Aufnahme eines Fadens auch hohl ausgebildet sein.

Bei einer zweiten bevorzugten Ausführungsform der erfindungsgemässen Vorrichtung sind in den beiden Haltern durchgehende Kanäle zur Aufnahme vorgespannter Stifte angeordnet, wobei ein Ende der Kanäle im Bereich der Ränder der Öffnungen mit der im wesentlichen gleichen Kontur liegt und die beiden Halter so aneinander anlegbar sind, dass sich die Kanäle paarweise gegenüberstehen und die in die Kanäle einsetzbaren Stifte mit beiden Haltern in Eingriff bringbar sind.

Zweckmässigerweise sind die Halter aus wenigstens zwei von einander lösbaren Halterteilen zusammengesetzt. Dadurch können die Halter nach dem Nähvorgang auf einfache Weise entfernt werden.

Die Öffnung im ersten Halter kann gegen die zur Auflage auf der Seitenwand des ersten Hohlorgans vorgesehene Seite erweitert sein, um die Beanspruchung und die damit mögliche Beschädigungsgefahr des Gewebes zu minimieren.

Der erste Halter kann zwischen zwei Niederhalterteilen eines Positionierarmes angeordnet sein, wobei die Niederhalterteile bevorzugt an einer Auflagefläche mit einer Unterdruckquelle verbindbare Saugöffnungen zum Festsaugen der Niederhalterteile an einer Gewebeunterlage aufweisen. Bei einer besonders bevorzugten Ausführungsform ist an jedem Niederhalterteil je ein Halterteil des ersten Halters festgelegt und die Niederhalterteile sind zur Trennung der Halterteile am Positionierarm schwenkbar angelenkt. Der erste Halter kann auch vollständig in einem Niederhalter integriert sein.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt schematisch in
- Fig. 1 eine Schrägsicht auf einen Positionierarm mit einem ersten Halter;
- Fig. 2 einen Querschnitt durch eine Koronararterie mit aufgesetztem ersten Halter;
- Fig. 3 den Querschnitt von Fig. 2 in einem weiteren Verfahrensstadium;
- Fig. 4 eine Schrägsicht auf die Anordnung von Fig. 3;
- Fig. 5 die Schrägsicht von Fig. 4 nach dem Wegschneiden überstehender Gefässteile;
- Fig. 6 eine Schrägsicht auf einen zweiteiligen zweiten Halter;
- Fig. 7 eine Seitenansicht des um eine Arterie gelegten Halters von Fig. 6;
- Fig. 8 die Anordnung von Fig. 7 aus einem anderen Blickwinkel;
- Fig. 9 - 10 das Aufsetzen des zweiten Halters auf den ersten Halter;

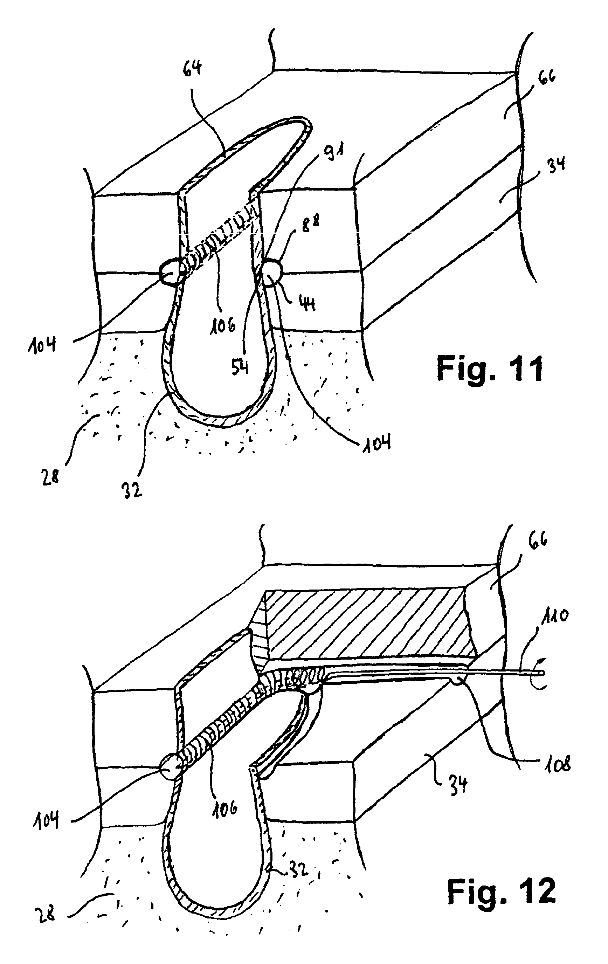

- Fig. 11 einen Schnitt durch die aufeinander liegenden ersten und zweiten Halter;
- Fig. 12 eine teilweise geschnittene Ansicht der Anordnung von Fig. 11;
- Fig. 13 einen Querschnitt durch die Anordnung von Fig. 11;
- Fig. 14 die Trennung der Halter nach erfolgter Anastomose;
- Fig. 15 eine erste Ausführungsform einer Helixnadel;
- Fig. 16 eine zweite Ausführungsform einer Helixnadel;
- Fig. 17 einen Schnitt durch den Randbereich der aufeinander liegenden ersten und zweiten Halter mit Verbindungsstiften;
- Fig. 18 den Schnitt von Fig. 17 nach der Trennung der Halter.

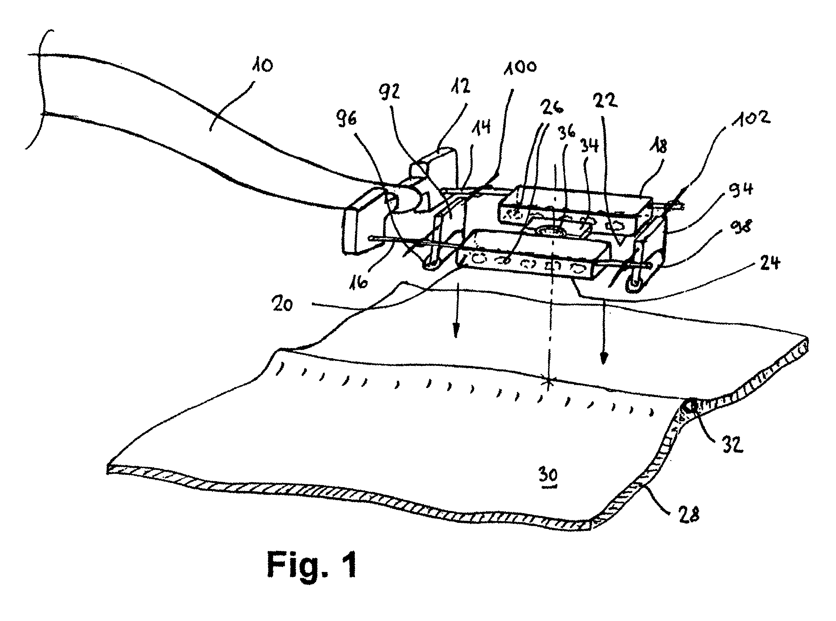

Ein in Fig. 1 dargestellter Teil einer Anordnung zur Durchführung einer Anastomose weist einen beweglichen Positionierarm 10 mit einer endständigen Montageplatte 12 auf. Von der Montageplatte 12 ragen zwei parallel zueinander angeordnete Montageachsen 14, 16 ab. An jeder Montageachse 14, 16 ist ein Niederhalterteil 18, 20 schwenkbar gelagert. An einer Auflagefläche 22, 24 der Niederhalterteile 18, 20 sind Saugöffnungen 26 angeordnet.

Die Beweglichkeit des beispielsweise an einem Rippenspreizer zu befestigenden Positionierarmes 10 ermöglicht die Ausrichtung der Montageplatte 12 mit den Niederhalterteilen 18, 20 bezüglich einer zur Durchführung der Anastomose vorgesehenen Koronararterie (Coronary Artery, CA) 32. Hierbei werden die beiden Niederhalterteile 18, 20 mit ihrer Anlagefläche 22, 24 so an die Oberfläche 30 der Herzwand 28 angelegt, dass die CA 32 parallel zu den Montageachsen 14, 16 mittig zwischen die beiden Niederhalterteile 18, 20 zu liegen kommt.

Die Niederhalterteile 18, 20 bzw. die Saugöffnungen 26 sind über in der Zeichnung nicht dargestellte Verbindungsleitungen an eine Unterdruckquelle angeschlossen. Der dadurch an den Saugöffnungen 26 erzeugte Unterdruck bewirkt, dass sich die Anlageflächen 22, 24 der Niederhalterteile 18, 20 an der Oberfläche 30 der Herzwand 28 festsaugen und so das zu verbindende Gefäss stabilisieren.

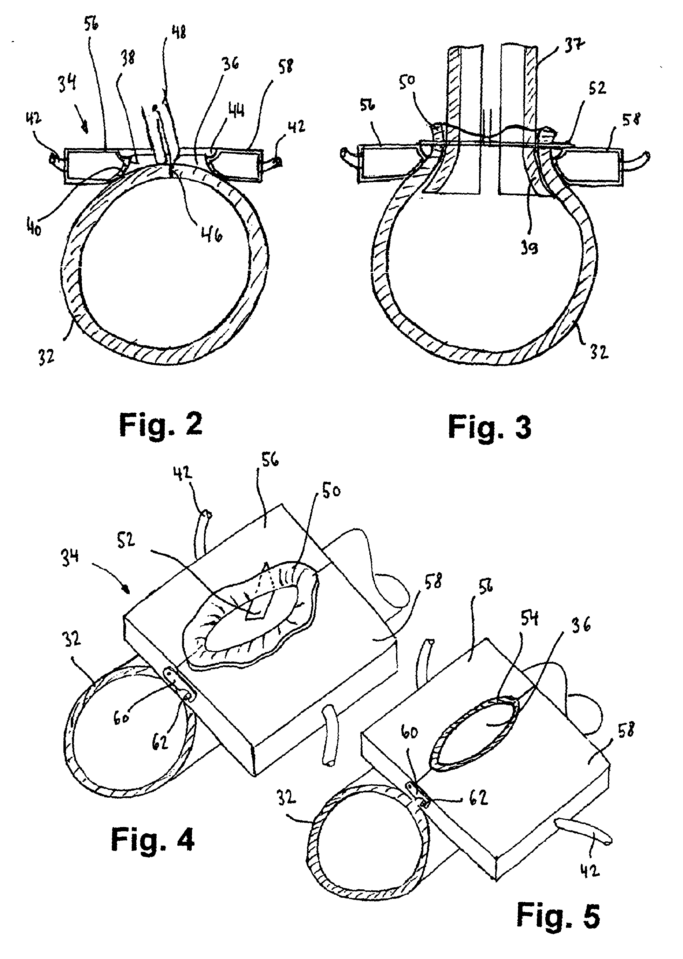

Zwischen den Niederhalterteilen 18, 20 ist ein plättchenförmiger erster Halter 34 mit einer diesen durchsetzenden Öffnung 36 angeordnet. Dieser erste Halter 34 ist entlang einer parallel zu den Montageachsen 14, 16 und senkrecht zwischen den beiden Niederhalterteilen 18, 20 durch die Mitte der Öffnung 36 verlaufenden Trennebene in zwei an je einem Niederhalterteil 18, 20 festgelegte Halterteile 56, 58 aufgeteilt (Fig. 2 bis 5). Der plättchenförmige erste Halter 34 ist hier quaderförmig ausgestaltet und weist beispielsweise eine Grundfläche von 15 x 15 mm und eine Dicke von 4 mm auf.

Zwischen den Montageachsen 14, 16 sind auf beiden Seiten der Niederhalterteile 18, 20 Klappen 92, 94 mit endständigen, quer zu den Montageachsen 14, 16 verlaufenden Druckrollen 96, 98 angeordnet. Die Klappen 92, 94 sind über je eine quer zu den Montageachsen 14, 16 stehende und an diesen festgelegte Querachse 100, 102 drehbar befestigt. Durch Schwenken der Klappen 92, 94 kann der Druck der auf der CA 32 aufliegenden Druckrollen 96, 98 und damit der Blutfluss durch die CA 32 in diesem Bereich eingestellt werden.

Wie in den Fig. 2 und 3 gezeigt, ist die Öffnung 36 im ersten Halter 34 gegen die zur Auflage auf der CA 32 vorgesehene Seite hin trompetenartig erweitert. Die derart gekrümmte Wand 38 weist über den Umfang verteilt angeordnete, über Saugleitungen 42 an eine in der Zeichnung nicht dargestellte Unterdruckquelle angeschlossene Saugöffnungen 40 auf. Nach einem durch die Öffnung 36 in der darunter liegenden CA 32 ausgeführten Schnitt 46 werden die an den Schnitt angrenzenden Gefässteile als Folge des an den Saugöffnungen 40 erzeugten Unterdruckes gegen die Öffnungswand 38 gesaugt und an dieser fixiert. Falls erforderlich können die Gefässteile im Schnittbereich zudem mit einer Pinzette 48 so weit aus der Öffnung 36 herausgezogen werden, dass überstehende Gefässteile 50 mit einem parallel zur Halteroberfläche geführten Messer 52 unter Ausbildung eines mit der Halteroberfläche bündigen Schnittrandes 54 abgetrennt werden können. Anstelle einer Pinzette eignet sich auch ein zweiteiliger Löffel 37 mit an die Krümmung der Öffnungswand 38 angepasster Löffelfläche 39 als Hebewerkzeug für die in die Öffnung 36 einzubringende Gewebefläche. Eine weitere Möglichkeit besteht darin, das Gewebe mit einer von oben durch die Öffnung 36 eingeführten Saugvorrichtung anzuheben.

Aus den Fig. 2 und 3 ergibt sich ohne weiteres die Vorteilhaftigkeit der sich gegen die CA 32 hin erweiternden Öffnung 36. Durch die Krümmung der Öffnungswand 38 verringert sich die Gefahr einer Gefässverletzung und die Auflagefläche für das Gefäss wird vergrössert. Zudem wird dessen Gleitfähigkeit an der Öffnungswand 38 verbessert. In den Fig. 2 und 3 ist ebenfalls erkennbar, dass der von der CA 32 weiter entfernt liegende Rand der Öffnung 36 als umlaufende Nut 44 mit kreisbogenförmigem Querschnitt ausgebildet ist. Die Funktion dieser umlaufenden Nut 44 ist weiter unten erläutert.

Wie aus den Fig. 4 und 5 hervorgeht, ergibt sich nach dem Wegschneiden des überstehenden Teils 50 der im ersten Halter 34 fixierten CA 32 ein mit der Halteroberfläche bündiger Schnittrand 54. Der Schnittrand 54 bzw. die Kontur der Öffnung 36 ist im wesentlichen oval oder ellipsenförmig.

Wie in den Fig. 4 und 5 dargestellt, besteht der erste Halter 34 aus zwei Halterteilen 56, 58. Die Halterteilen 56, 58 berühren sich in einer die Öffnung 36 halbierenden Trennebene und können in dieser Stellung über einen am ersten Halterteil 56 angeordneten seitlichen Verschlusshaken 60 und einen seitlich am zweiten Halterteil 58 angeordneten Verschlussstift 62 in der zusammengebauten Lage fixiert werden.

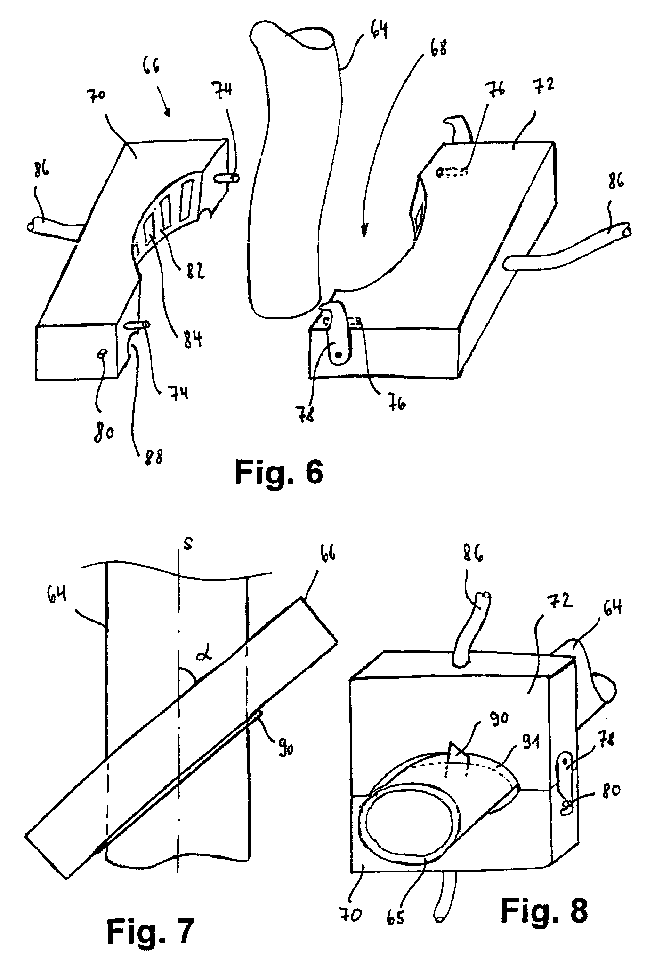

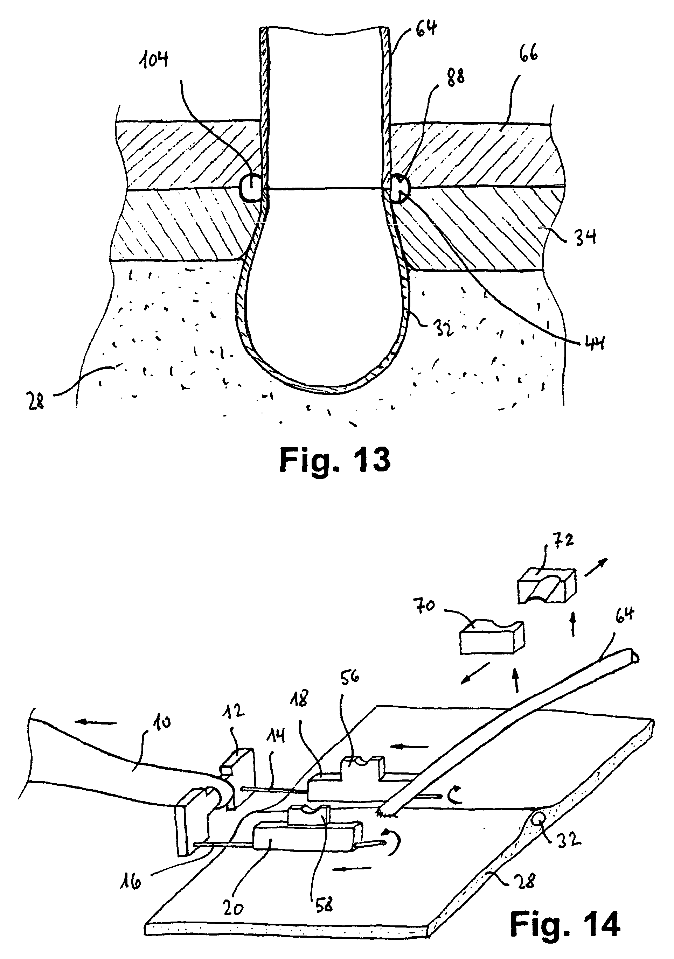

In den Fig. 6 bis 8 ist ein mit dem ersten Halter 34 vergleichbarer zweiter Halter 66 dargestellt. Der aus zwei Halterteilen 70, 72 bestehende zweite Halter 66 ergibt im zusammengesetzten Zustand ebenfalls ein quaderförmiges Plättchen mit einer Grundfläche von beispielsweise 15 x 15 mm und einer Höhe von beispielsweise 8 mm. Im zusammengebauten Zustand weist der zweite Halter 66 eine Öffnung 68 auf. Die Wand 82 der Öffnung 68 weist eine zylindrische Form auf, und die Zylinderachse s der Öffnungswand 82 schliesst mit der Halterebene einen Winkel α von etwa 45° ein (Fig. 7). Dieser Winkel entspricht dem nach der Anastomose zwischen der CA 32 und der LIMA 64 gewünschten Winkel (Fig. 14).

Mit dem zweiten Halter 66 wird der Stumpf eines Hohlorgans 64 mit einem im wesentlichen - kreisförmigen Querschnitt, im vorliegenden Beispiel die für die Versorgung der linken Brusthälfte mit arteriellem Blut verantwortliche Arterie, die sogenannte Left Internal Mammary Artery (LIMA) umschlossen. Hierbei werden die beiden Halterteile 70, 72 so um die LIMA 64 gelegt, dass diese zwischen den beiden Halterteilen 70, 72 eingeschlossen ist. Der Halterteil 70 weist von zwei Stossflächen abragende Führungsstifte 74 auf, die im zusammengebauten Zustand des zweiten Halters 66 in entsprechende Führungsbohrungen 76 im anderen Halterteil 72 eingreifen. In dieser Stellung können die beiden Halterteile 70, 72 über seitlich an diesen angeordnete Verschlusshaken 78 und Verschlussstifte 80 fixiert werden.

Wie in Fig. 6 gezeigt, sind an der Öffnungswand 82 über den Umfang verteilte Saugöffnungen 84 angeordnet. Jedes Halterteil 70, 72 ist als Hohlkörper ausgebildet, welcher über Saugleitungen 86 an eine Unterdruckquelle angeschlossen ist. Der an den Saugöffnungen 84 erzeugte Unterdruck bewirkt, dass die LIMA 64 gegen die Öffnungswand 82 gesaugt und an dieser in ihrer Lage fixiert wird. Der überstehende Teil 65 des Stumpfes der LIMA 64 wird gemäss Fig. 8 mit einem Messer 90 weggeschnitten, so dass sich ein mit der Oberfläche des zweiten Halters 66 bündiger Schnittrand 91 ergibt. Dieser Schnittrand 91 der LIMA 64 bzw. der an den Schnittrand 91 angrenzende Rand der Öffnung 68 weist eine mit dem Schnittrand 54 der CA 32 bzw. mit dem an den Schnittrand 54 angrenzenden Rand der Öffnung 36 im ersten Halter 34 identische Form auf.

Wie aus Fig. 6 hervorgeht, ist auch der an den Schnittrand 91 angrenzende Rand der Öffnung 68 des zweiten Halters 66 als umlaufende Nut 88 mit kreisbogenförmigem Querschnitt ausgebildet. Die Funktion dieser umlaufenden Nut 88 ist weiter unten erläutert.

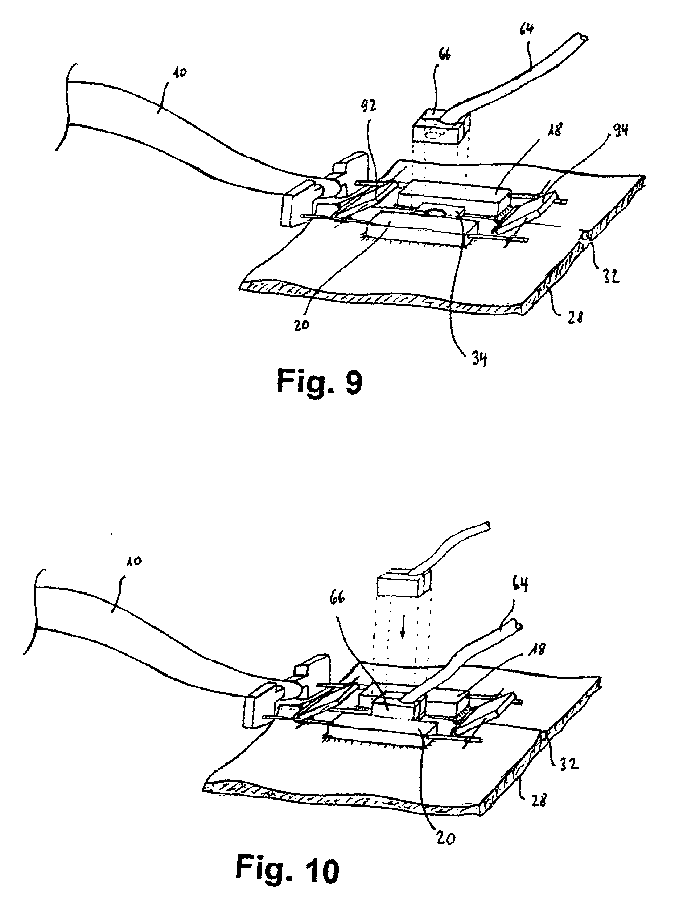

Wie in den Fig. 9 und 10 dargestellt, wird der zweite Halter 66 mit in diesem fixierter LIMA 64 über dem ersten Halter 34 mit in diesem fixiertem Teil der CA 32 positioniert. Der zweite Halter 66 wird nun an den ersten Halter 34 herangeführt, bis sich die beiden Halter 34, 66 berühren und der Schnittrand 91 der LIMA 64 dem Schnittrand 54 der CA 32 deckungsgleich anliegt.

In dieser Lage ergänzen sich die umlaufenden Nuten 44, 88 der beiden Halter 34, 66 zu einer gemeinsamen Führungsnut 104 für eine Helixnadel 106, welche tangential über seitliche Hilfsnuten 108 in die Führungsnut 104 eingeführt werden kann. Am rückwärtigen Ende der Helixnadel 106 ist eine Achse 110 befestigt, welche beispielsweise über einen in der Zeichnung nicht dargestellten Elektromotor in eine Drehbewegung versetzt werden kann. Bei jedem Umgang der Helixnadel 106 in der Führungsnut 104 erfolgt ein Durchstechen der CA 32 und der LIMA 64 im Bereich der sich auf der Höhe der Öffnung der Führungsnut 104 berührenden Schnittränder 54, 91. Durch die kontinuierliche Drehbewegung der Achse 110 "schraubt" sich die Helixnadel 106 durch das Gewebe.

Zur Durchführung eines vollständigen Nähvorganges werden üblicherweise zwei Helixnadeln 106 verwendet. Jede der Helixnadeln 106 wird über je eine seitliche Hilfsnut 108 tangential in die Führungsnut 104 eingeführt und ihre Spitze 107 nach dem Durchlaufen je eines halben Umfanges wiederum tangential aus der Führungsnut 104 heraus in je eine weitere Hilfsnut 108 eingeführt.

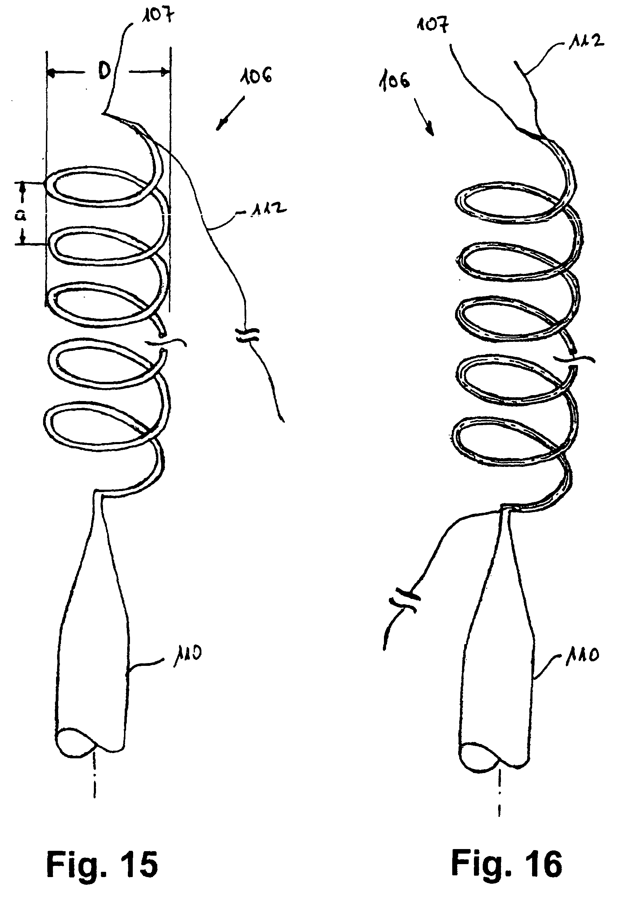

Ein von der Helixnadel 106 mitgeführter Faden 112 führt zur gewünschten Nähverbindung. Der Faden 112 kann an der Spitze 107 der Helixnadel 106 befestigt und von der Helixnadel 106 nachgezogen werden (Fig. 15). Eine andere Möglichkeit besteht darin, den Faden 112 in einer hohlen Helixnadel 106 mitzuführen (Fig.16). Beim Austritt der Nadelspitze 107 aus einer tangential zur Führungsnut 104 angeordneten Hilfsnut 108 wird der Faden 112 gefasst und die Helixnadel 106 anschliessend mit umgekehrter Drehrichtung rückwärts aus der Führungsnut 106 herausgedreht. Eine Nähverbindung kann aber auch ohne Faden allein mit einer Helixnadel hergestellt werden. In diesem Fall bleibt die Helixnadel selbst als "Faden" im Gewebe zurück.

Bei jeder mit einer Helixnadel 106 ausgeführten Nähverbindung kann der Stichabstand und die Stichhöhe durch entsprechende Wahl der Steigung a und des Durchmessers D der Helixnadel 106 vorgegeben werden.

Es versteht sich von selbst, dass die Enden der im Gewebe zurückbleibenden Fäden gegen Herausrutschen gesichert werden müssen. Bekannte Sicherungen sind beispielsweise Knoten, Clipse, Briden, Klemmen und Kleb- bzw. Schweissverbindungen.

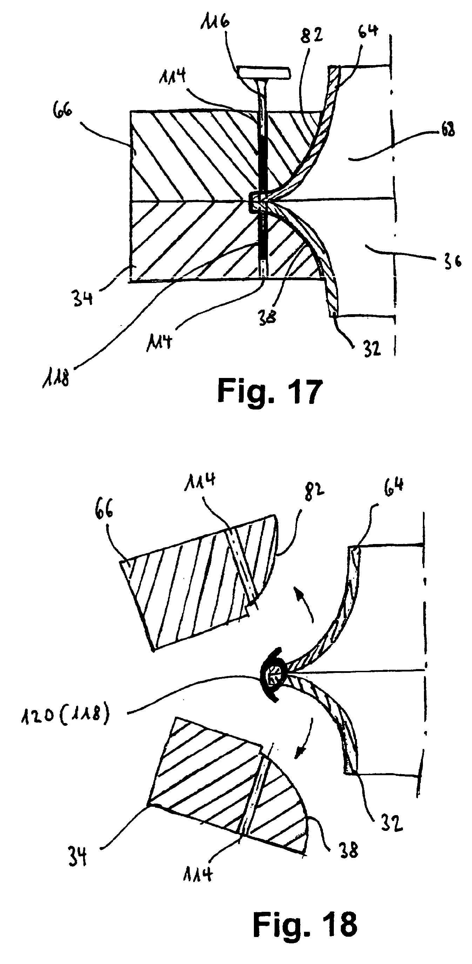

Eine weitere Möglichkeit, zwei Gefässteile mit einander zu verbinden, ist in Fig.17 und 18 dargestellt. Die Öffnungswand 38, 82 in den beiden Haltern 34, 66 ist hier so ausgebildet, dass die freien Ränder der mit einander zu verbindenden CA 32 und LIMA 64 radial nach aussen gerichtet sind und einander flanschartig anliegen. Im Randbereich der Öffnungen 36, 68 sind über den Umfang verteilte, die beiden Halter 34, 66 durchsetzende Kanäle 114 angeordnet, in welche beispielsweise mittels eines pneumatischen Zylinders 116 Stifte 118 eingesetzt werden können. Anstelle des pneumatischen Zylinders 116 können auch hydraulische Kräfte eingesetzt werden, mit denen die Stifte 118 durch das Gewebe bewegt werden können. Die hydraulischen Kräfte können beispielsweise durch Wasserdruck auf die Stifte 118 übertragen werden, d.h. die Stifte 118 können mit Wasserdruck durch das Gewebe geführt werden. Damit ist ein äusserst platzsparender Antrieb möglich, was für minimal invasive Operationen von grosser Bedeutung ist. Gleichzeitig ist das energieübertragende Medium Wasser bei geeigneter Vorbereitung steril und körperverträglich. Zudem ist die Energiezufuhr problemlos von aussen möglich.

Die in den Kanälen 114 unter einer Vorspannung stehenden Stifte 118 bilden in ihrem ungespannten Zustand geschlossene Schlaufen 120 mit sich kreuzenden Enden.

Die in die Kanäle 114 eingesetzten Stifte 118 durchdringen die einander flanschartig anliegenden Ränder der CA 32 und der LIMA 64. Nach dem Einsetzen der Stifte 118 werden die beiden Halter 34, 66 von einander getrennt. Dadurch werden die Stifte 118 freigelegt und nehmen wieder ihre ursprüngliche Form einer geschlossenen Schlaufe 120 an. Die über den Umfang der mit einander zu verbindenden Gefässränder verteilten Schlaufen 120 führen zu einer dauerhaften Verbindung der CA 32 und der LIMA 64.

Nach Beendigung des Nähvorganges werden die Halterteile 56, 58 des ersten Halters 34 und die Halterteile 70, 72 des zweiten Halters 66 von einander getrennt, wie dies in Fig. 14 schematisch dargestellt ist. Die Trennung der an den Niederhalterteilen 18, 20 festgelegten Halterteile 56, 58 des ersten Halters 34 erfolgt hier durch eine 90°-Drehung der Niederhalterteile 18, 20 um die Montageachsen 14, 16.

## Patentansprüche

1. Vorrichtung zur Durchführung einer Anastomose mit einer Nähverbindung zwischen einer Seitenwand oder einem endständigen Stumpf eines ersten Hohlorgans (32) und einer Seitenwand oder einem endständigen Stumpf eines zweiten Hohlorgans (64),
**gekennzeichnet durch**
- einen ersten Halter (34) mit einer diesen durchsetzenden, von einer Wand (38) mit Saugöffnungen (40) begrenzten Öffnung (36) zur Aufnahme und Fixierung eines Seitenwandbereiches oder eines endständigen Stumpfes des ersten Hohlorgans (32) und
- einen zweiten Halter (66) mit einer diesen durchsetzenden, von einer Wand (82) mit Saugöffnungen (84) begrenzten Öffnung (68) zur Aufnahme und Fixierung eines Seitenwandbereiches oder eines endständigen Stumpfes des zweiten Hohlorgans (64),
wobei
- die Saugöffnungen (40, 84) mit einer Unterdruckquelle verbindbar sind,
- die Öffnungen (36, 68) auf je einer Seite der Halter (34, 66) eine im wesentlichen gleiche Kontur aufweisen,
- im Bereich der Ränder der Öffnungen (36, 68) mit der im wesentlichen gleichen Kontur Führungen für wenigstens ein Verbindungselement angeordnet sind und
- die beiden Halter (34, 66) so aneinander anlegbar sind, dass sich die Öffnungen (36, 68) mit der im wesentlichen gleichen Kontur zur Herstellung der Nähverbindung im wesentlichen deckungsgleich gegenüberstehen.

2. Vorrichtung nach Anspruch 1, **dadurch gekennzeichnet, dass** die Ränder der Öffnungen (36, 68) mit der im wesentlichen gleichen Kontur als umlaufende Nuten (44, 88) mit kreisbogenförmigem Querschnitt ausgebildet sind und die beiden Halter (34, 66) so aneinander anlegbar sind, dass sich die umlaufenden Nuten (44, 88) gegenüberstehen und eine Führungsnut (104) für eine Helixnadel (106) bilden.

3. Vorrichtung nach Anspruch 2, **dadurch gekennzeichnet, dass** am rückwärtigen Ende der Helixnadel (106) eine Achse (110) zur Verbindung mit einem Antriebsaggregat zur automatischen Ausführung einer Drehbewegung festgelegt ist.

4. Vorrichtung nach Anspruch 1, **dadurch gekennzeichnet, dass** in den beiden Haltern (34, 66) durchgehende Kanäle (114) zur Aufnahme vorgespannter Stifte (118) angeordnet sind, wobei ein Ende der Kanäle (114) im Bereich der Ränder der Öffnungen (36, 68) mit der im wesentlichen gleichen Kontur liegt und die beiden Halter (34, 66) so aneinander anlegbar sind, dass sich die Kanäle (114) paarweise gegenüberstehen und die in die Kanäle (114) einsetzbaren Stifte (118) mit beiden Haltern (34, 68) in Eingriff bringbar sind.

5. Vorrichtung nach einem der Ansprüche 1 bis 4, **dadurch gekennzeichnet, dass** die Halter (34,66) aus wenigstens zwei von einander lösbaren Halterteilen (56, 58; 70, 72) zusammengesetzt sind.

6. Vorrichtung nach einem der Ansprüche 1 bis 5, **dadurch gekennzeichnet, dass** die Öffnung (36) im ersten Halter (34) gegen die zur Auflage auf der Seitenwand des ersten Hohlorgans (32) vorgesehenen Seite erweitert ist.

7. Vorrichtung nach einem der Ansprüche 1 bis 6, **dadurch gekennzeichnet, dass** der erste Halter (34) zwischen zwei Niederhalterteilen (18, 20) eines Positionierarmes (10) angeordnet ist.

8. Vorrichtung nach Anspruch 7, **dadurch gekennzeichnet, dass** die Niederhalterteile (18, 20) an einer Auflagefläche (22, 24) mit einer Unterdruckquelle verbindbare Saugöffnungen (26) zum Festsaugen der Niederhalterteile (18, 20) an einer Gewebeunterlage (28) aufweisen.

9. Vorrichtung nach Anspruch 7 oder 8, **dadurch gekennzeichnet, dass** an jedem Niederhalterteil (18, 20) je ein Halterteil (56, 58) des ersten Halters (34) festgelegt ist und die Niederhalterteile (18, 20) zur Trennung der Halterteile (56, 58) am Positionierarm (10) schwenkbar angelenkt sind.

## Revendications

1. Dispositif pour pratiquer une anastomose avec une suture cousue entre une paroi latérale ou un moignon d'extrémité d'un premier organe creux (32) et une paroi latérale ou un moignon d'extrémité d'un deuxième organe creux (64),
**caractérisé par**
- un premier support (34) avec une ouverture (36) traversant celui-ci et limitée par une paroi (38) avec des ouvertures d'aspiration (40), destiné à recevoir et à fixer une zone de paroi latérale ou un moignon d'extrémité du premier organe creux (32), et
- un deuxième support (66) avec une ouverture (68) traversant celui-ci et limitée par une paroi (82) avec des ouvertures d'aspiration (84), destiné à recevoir et à fixer une zone de paroi latérale ou un moignon d'extrémité du deuxième organe creux (64),
dans lequel
- les ouvertures d'aspiration (40, 84) peuvent être raccordées à une source de dépression,
- les ouvertures (36, 68) présentent sur chaque face des supports (34, 66) un contour sensiblement identique,
- des guides pour au moins un élément de liaison sont disposés dans la région des bords des ouvertures (36, 68) avec le contour sensiblement identique, et
- les deux supports (34, 66) peuvent être posés l'un sur l'autre de telle façon que les ouvertures (36, 68) avec le contour sensiblement identique se recouvrent sensiblement à l'identique pour la réalisation de la suture.

2. Dispositif selon la revendication 1, **caractérisé en ce que** les bords des ouvertures (36, 68) avec le contour sensiblement identique sont réalisés sous forme de rainures périphériques (44, 88) de section transversale en arc de cercle et les deux supports (34, 66) peuvent être superposés de telle façon que les rainures périphériques (44, 88) se trouvent en face l'une de l'autre et forment une rainure de guidage (104) pour une aiguille hélicoïdale (106).

3. Dispositif selon la revendication 2, **caractérisé en ce qu'**un axe (110) est fixé à une extrémité arrière de l'aiguille hélicoïdale (106) pour le raccordement à un ensemble d'entraînement en vue de l'exécution automatique d'un mouvement de rotation.

4. Dispositif selon la revendication 1, **caractérisé en ce que** des canaux continus (114) sont formés dans les deux supports (34, 66) pour recevoir des broches précontraintes (118), une extrémité des canaux (114) étant située dans la région des bords des ouvertures (36, 68) avec le contour sensiblement identique et les deux supports (34, 66) pouvant être superposés de telle façon que les canaux (114) soient disposés par paires les uns en face des autres et que les broches (118) insérables dans les canaux (114) puissent être mises en prise avec les deux supports (34, 68).

5. Dispositif selon l'une quelconque des revendications 1 à 4, **caractérisé en ce que** les supports (34, 66) se composent d'au moins deux parties de support (56, 58; 70, 72) séparables l'une de l'autre.

6. Dispositif selon l'une quelconque des revendications 1 à 5, **caractérisé en ce que** l'ouverture (36) dans le premier support (34) est élargie vers le côté prévu pour l'appui sur la paroi latérale du premier organe creux (32).

7. Dispositif selon l'une quelconque des revendications 1 à 6, **caractérisé en ce que** le premier support (34) est disposé entre deux parties de patin (18, 20) d'un bras de positionnement (10).

8. Dispositif selon la revendication 7, **caractérisé en ce que** les parties de patin (18, 20) présentent sur une face d'appui (22, 24) des ouvertures d'aspiration (26) pouvant être raccordées à une source de dépression pour aspirer les parties de patin (18, 20) sur un appui du tissu (28).

9. Dispositif selon la revendication 7 ou 8, **caractérisé en ce qu'**une partie de support (56, 58) du premier support (34) est fixée à chaque partie de patin (18, 20) et les parties de patin (18, 20) sont articulées de façon pivotante sur le bras de positionnement (10) pour la séparation des parties de support (56, 58).

## Claims

1. Device for performing anastomosis, with a suture connection between a side wall or end stump of a first hollow organ (32) and a side wall or end stump of a second hollow organ (64), **characterized by**
- a first holder (34) with an opening (36) which passes through it, is delimited by a wall (38) with suction openings (40) and is used for receiving and fixing a side wall area or end stump of the first hollow organ (32), and
- a second holder (66) with an opening (68) which passes through it, is delimited by a wall (82) with suction openings (84) and is used for receiving and fixing a side wall area or end stump of the second hollow organ (64),
where
- the suction openings (40, 84) can be connected to a partial vacuum source,
- the openings (36, 68) have a substantially identical contour on each side of the holders (34, 66),
- guides for at least one connecting element are arranged in the area of the edges of the openings (36, 68) with the substantially identical contour, and
- the two holders (34, 66) can be placed on one another such that the openings (36, 68) with the substantially identical contour lie substantially congruently opposite one another in order to produce the suture connection.

2. Device according to Claim 1, **characterized in that** the edges of the openings (36, 68) with the substantially identical contour are designed as circumferential grooves (44, 88) with a cross section in the shape of an arc of a circle, and the two holders (34, 66) can be placed on one another in such a way that the circumferential grooves (44, 88) lie opposite one another and form a guide groove (104) for a helix needle (106).

3. Device according to Claim 2, **characterized in that** a shaft (110) for connection to a drive unit, to permit automatic execution of a rotation movement, is secured on the rear end of the helix needle (106).

4. Device according to Claim 1, **characterized in that** continuous channels (114) for receiving pretensioned pins (118) are arranged in the two holders (34, 66), where one end of the channels (114) lies in the area of the edges of the openings (36, 68) with the substantially identical contour and the two holders (34, 66) can be placed on one another in such a way that the channels (114) lie opposite one another in pairs and the pins (118), which can be inserted into the channels (114), can be brought into engagement with the two holders (34, 68).

5. Device according to one of Claims 1 to 4, **characterized in that** the holders (34, 66) are made up of at least two holder parts (56, 58; 70, 72) that can be detached from one another.

6. Device according to one of Claims 1 to 5, **characterized in that** the opening (36) in the first holder (34) is widened towards the side intended to bear on the side wall of the first hollow organ (32).

7. Device according to one of Claims 1 to 6, **characterized in that** the first holder (34) is arranged between two holding-down parts (18, 20) of a positioning arm (10).

8. Device according to Claim 7, **characterized in that** the holding-down parts (18, 20) have, on a bearing surface (22, 24), suction openings (26) which can be connected to a partial vacuum source for the purpose of sucking the holding-down parts (18, 20) firmly onto a tissue base (28).

9. Device according to Claim 7 or 8, **characterized in that** one holder part (56, 58) of the first holder (34) is in each case secured on each holding-down part (18, 20), and the holding-down parts (18, 20) are articulated pivotably on the positioning arm (10) for separating the holder parts (56, 58).
